(19) Europäisches Patentamt
European Patent Office
Office européen des brevets

(11) EP 0 722 938 B1

(12) **EUROPÄISCHE PATENTSCHRIFT**

(45) Veröffentlichungstag und Bekanntmachung des Hinweises auf die Patenterteilung:
**10.05.2000 Patentblatt 2000/19**

(51) Int Cl.[7]: **C07D 249/20**, C08K 5/3475

(21) Anmeldenummer: **95116447.4**

(22) Anmeldetag: **19.10.1995**

(54) **Verfahren zur Herstellung eines radikalisch polymerisierbaren UV-Absorbers auf Basis Benz-triazolylphenol**

Process for preparation of a radically polymerizable UV absorber based on benztriazolyl phenols

Procédé pour la préparation d'un absorbant UV, réticulable par radicaux, à base de benzotriazolyle-phénol

(84) Benannte Vertragsstaaten:
**CH DE FR GB IT LI**

(30) Priorität: **28.10.1994 DE 4438575**

(43) Veröffentlichungstag der Anmeldung:
**24.07.1996 Patentblatt 1996/30**

(73) Patentinhaber: **Röhm GmbH**
**64293 Darmstadt (DE)**

(72) Erfinder:
- **Arndt, Peter Joseph, Dr.**
**D-64342 Seeheim-Jugenheim (DE)**
- **Sander, Ingo Bernd**
**D-64579 Gernsheim (DE)**
- **Scharf, Jakob**
**D-67549 Worms (DE)**
- **Riemann, Karl Heinz**
**D-64665 Alsbach (DE)**

(56) Entgegenhaltungen:
**EP-A- 0 474 595**
**CH-A- 408 033**
**FR-A- 2 601 365**
**US-A- 3 399 173**
**US-A- 4 652 656**

- **PATENT ABSTRACTS OF JAPAN vol. 12, no. 496 (C-555), 23.Dezember 1988 & JP-A-63 205334 (ADEKA ARGUS CHEM CO LTD), 24.August 1988,**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 722 938 B1

## Beschreibung

Gebiet der Erfindung

Die Erfindung betrifft den UV-Schutz von Kunststoffmassen, z.B. Kunststoffplatten, insbesondere ein vereinfachtes und umweltfreundliches Verfahren zur Herstellung von radikalisch polymerisierbaren UV-Absorbern auf Basis Benztriazolylphenol, wobei in hoher Ausbeute Produkte von geringer Eigenfarbe erhalten werden.

Stand der Technik

Bei der Kunststoffverarbeitung stören flüchtige Beimischungen, z.B. ein flüchtiger (=nicht sublimierfester) UV-Absorber. Um dessen Flüchtigkeit zu reduzieren bzw. zu unterbinden gibt es prinzipiell zwei Möglichkeiten: Sein Molekulargewicht zu vergrößern, z.B. zu verdoppeln, oder eine polymerisierbare Gruppe einzubauen und kovalent mit dem Kunststoff zu verbinden. Bei UV-Absorbern auf Basis Benztriazol sind beide Modifikationen durch ein und dieselbe Kondensationsreaktion herstellbar. Bevorzugt wird in der vorliegenden Erfindung die zweite Möglichkeit.

Besonders wirksame UV-Absorber sind Derivate des Benztriazolylphenols (II). Sie werden bevorzugt aus schwefelsaurer Lösung mit Formaldehyd oder einem Formaldehydderivat kondensiert. Eine andere Methode der Kondensation, nämlich die in Gegenwart eines sekundären Amins, bekannt als Mannich-Kondensation, hat Nachteile: Sie ist mehrstufig, erfordert also einen größeren Arbeitsaufwand und birgt außerdem die Gefahr, daß die Produkte durch geringe Mengen an Aminen verunreinigt werden. Diese beeinträchtigen Farbe und Witterungsverhalten. Das erfindungsgemäße Herstellverfahren beruht daher auf der einstufigen, schwefelsauren Kondensation.

Für die Herstellung eines radikalisch polymerisierbaren UV-Stabilisators nach dem Verfahren der Kondensation in Schwefelsäure ist das US-Patent 4 652 656 der nächste Stand der Technik. Hier wird das Benzotriazolylphenol-Derivat mit dem Formaldehyd-Derivat von Methacrylamid, dem N-Methylolmethacrylamid, kondensiert. Letzteres wird in fester Form eingesetzt. Reaktionstemperatur ist 15-20°C. Die Fällung des Kondensationsprodukts erfolgt in einem Überschuß an Eiswasser. Um ein reines, farblich einwandfreies Endprodukt zu erhalten, kann auf die Verwendung von organischen Lösungsmitteln nicht verzichtet werden: Das Fällungsprodukt wird in Methylenchlorid gelöst, mit Wasser ausgeschüttelt und nach Abzug des Lösungsmittels mit Hexan und Methanol gewaschen.

Das französische Patent FR-A-2601365 beschreibt radikalisch polymerisierbare UV-Stabilisatoren, die vom Acrylamid abgeleitet sind und hergestellt werden, indem zuerst eine säurekatalysierte Kondensationsreaktion und anschließend eine Aufarbeitung der Reaktionsmischung durch Eingießen in Eiswasser durchgeführt wird. Nach der Isolation des Kondensationsprodukts ist eine weitere Reinigung durch Umkristallisation erforderlich.

Gegenstand des schweizerischen Patentes CH-A-408033 ist ein Verfahren zur Herstellung von substituierten 2-(2-Hydroxyphenyl)-benztriazolverbindungen, wobei entsprechend substituierbare 2-(2-Hydroyphenyl)-benztriazolverbindungen mit der N-Methylolverbindung eines entsprechenden Carbonsäureamids oder mit einem wie diese Methylolverbindung reagierenden funktionellen Derivat derselben in Gegenwart saurer Kondensationsmittel umgesetzt werden. Die Kondensationsreaktion erfolgt bei Raumtemperatur, gegebenenfalls auch bei erhöhter Temperatur bis zu 80 °C. Die Reaktionsprodukte werden durch Eingießen der schwefelsauren Lösungen oder Suspensionen in Eiswasser abgeschieden, durch Abfiltrieren oder Abschleudern isoliert und anschließend durch Umkristallisieren aus organischen Lösungsmitteln weiter gereinigt.

Zum chemischen Aufbau von UV-Absorbern auf Basis Benztriazol besteht ein breiter Stand der Technik. Variationsmöglichkeiten sind in dem oben genannten Schutzrecht beschrieben. Eine umfassendere Betrachtung der Variationsmöglichkeiten ist in der EPA 0 010 518, sowie in der DE-OS 4118545 enthalten.

Aufgabe

Ein entscheidendes Qualitätskriterium für die Verwendung von UV-Absorbern in Kunststoffen ist eine möglichst geringe Eigenfärbung. Sie wird durch die "Farbzahl" ausgedrückt. Dabei wird die Extinktionsdifferenz des UV-Absorbers in einer definierten Lösung (z. B. in 2 %iger Lösung in Methylmethacrylat) bei Wellenlängen von 460 und 620 nm bestimmt. Die Farbzahl kann Werte von 0 - 500 annehmen und wird über einen Platin-Kobalt-Standard geeicht.

Ein Ziel der vorliegenden Erfindung ist es, ein Verfahren zur Herstellung sublimierfester UV-Absorber mit einer niedrigen Farbzahl anzubieten.

Ein bevorzugtes Ziel der vorliegenden Erfindung ist es, das Verfahren möglichst umweltfreundlich zu gestalten, vor allem die Verwendung von organischen Lösungsmitteln zu vermeiden, die allesamt schwierig zu entsorgen sind. Die Vermeidung von Lösungsmitteln schließt auch eine verbesserte Wirtschaftlichkeit mit ein.

Schließlich ist es ein Ziel der vorliegenden Erfindung, hohe Ausbeuten bezogen auf die Ausgangsstoffe zu erreichen.

Lösung

Die Aufgaben werden durch ein neues Herstellungsverfahren gelöst, das in den Kondensationsbedingungen, vor allem aber bei der Isolierung des Kondensats entscheidend vom bisherigen Stand der Technik abweicht.

Es ist ein Verfahren zur Herstellung eines schwerflüchtigen bzw. radikalisch polymerisierbaren UV-Absorbers der allgemeinen Formel

$$R' - CH_2 - R'' \qquad (I)$$

wobei R' gleich

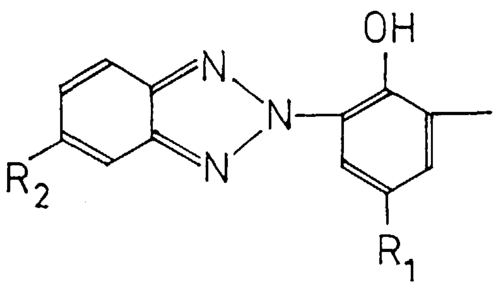

$R_1$ gleich H, $C_1$ - $C_{12}$-Alkyl, $C_6$ - $C_{10}$-Aryl,
$R_2$ gleich H, Halogen, $C_1$ - $C_{12}$-Alkyl, $C_6$ - $C_{10}$-Aryl ist und
R'' gleich

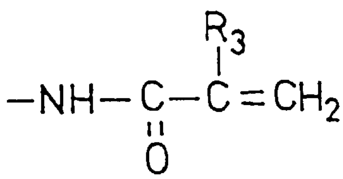

und $R_3$ gleich H oder $CH_3$ ist, durch Kondensation eines Benztriazolylphenols (II)

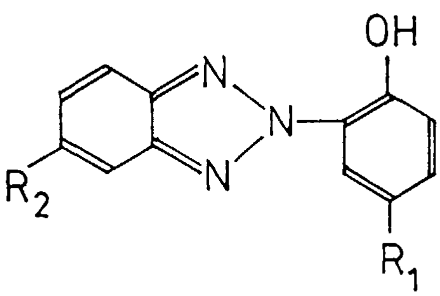

mit N-Methylol(meth)acrylamid (III) in homogener Phase in Schwefelsäure in einer Stufe
dadurch gekennzeichnet, daß

a) die Kondensationsreaktion bei 0-10°C durchgeführt wird
b) die Fällung des in Schwefelsäure gelösten Kondensats durch Eintrag in einen Überschuß an Wasser von über 80°C erfolgt.

Die Kondensationsreaktion verläuft folgendermaßen:

$$(II) + (III) = R' - CH_2 - R'' + H_2O$$

Die wichtigsten Stufen der Herstellung in Übersicht sind:

1. Kondensationsreaktion des Benztriazolylphenols mit dem Formaldehydderivat in konzentrierter Schwefelsäure
2. Fällung des entstandenen Kondensats in Wasser
3. Isolierung und Reinigung des Kondensats
4. Trocknung zum Endprodukt

Es wurde gefunden, daß bei Durchführung der Kondensationsreaktion von Benztriazolylphenolen in konzentrierter Schwefelsäure mit Formaldehydderivaten bei Temperaturen von 0 °C bis +10 °C, bevorzugt bei 2 bis 5 °C besonders niedrige Farbzahlen im Endprodukt erzielt werden. Die niedrige Temperatur ist ein wichtiges Merkmal der Erfindung.
Der Wassergehalt im Reaktionsansatz soll nicht höher als 20 % sein, da sonst die Kondensationsreaktion merklich langsamer abläuft. Bevorzugt sind Wassergehalte von < 10 %, besonders bevorzugt < 5 % im Gesamtansatz. Vollständig wasserfrei kann nur mit großem Aufwand gearbeitet werden, denn die technisch erhältliche konzentrierte Schwefelsäure ist ca. 98 %ig, enthält also schon ca. 2 % Wasser. Meist bringt auch das Kondensationsreagens, wie z.B. N-Methylolmethacrylamid herstellungsbedingt bereits eine gewisse Menge Wasser mit. Es liegt z.B. 70-80 %ig vor.
Das Kondensationsmedium, die konzentrierte Schwefelsäure, wird im Überschuß vorgelegt. Sie kann z.B. als 10 bis 100 facher molarer Überschuß gemessen am eingesetzten Kondensationsreagens vorliegen. Die molaren Verhältnisse Benztriazolderivat : Kondensationsreagens werden im Bereich 1:1 bis 1:2 eingestellt, wobei man vorteilhaft das billigere Reagens, nämlich das Aldehyreagens im Überschuß einsetzt. Bewährt haben sich Molverhältnisse Benztriazolderivat : N-Methylol (meth)acrylamid = 1:1.1 bis 1:1.6.

Ein entscheidendes Kennzeichen der vorliegenden Erfindung ist der ungewöhnliche Isolierungsschritt für die Gewinnung des Kondensats.
Nach dem Stand der Technik erfolgt die Isolierung des Reaktionsgemisches durch Fällung mit Eiswasser, einer altbewährten Methode. Erfindungsgemäß wird aber mit Wasser von über 80 °C, bevorzugt sogar mit siedendem Wasser gefällt. Dabei wird das in Schwefelsäure gelöste Kondensat in siedendes Wasser langsam, d.h. innerhalb von ca 0.3 bis 3 Stunden, eingetragen. Die Siedetemperatur steigt dabei mit zunehmendem Schwefelsäuregehalt an. Sie erreicht z.B. zuletzt 103 °C, wenn das Fällmedium ca 30 %ig an Schwefelsäure ist. Im geschlossenen Kessel kann auch unter Über- oder Unterdruck gearbeitet werden, wobei Temperaturen z.B von 80 bis 120 °C möglich sind. Vorzugsweise arbeitet man aber unter Atmosphärendruck. Das vorgelegte, siedende Wasser liegt im Überschuß vor. Bevorzugt ist ein 1.5 bis 3 facher Überschuß bezogen auf das Gewicht des einzutragenden Kondensationsansatzes. Temperaturen unter 80 °C führen zu einem verschlechterten Fällergebnis.

Überraschend wurde gefunden, daß unter obigen Bedingungen der hohen Temperatur bei der Fällung das Produkt besonders grobteilig anfällt, dadurch gut filtrierbar und auswaschbar, und vor allem von hoher Reinheit ist. Dadurch braucht man nicht mehr mit organischen Lösungsmitteln nachzureinigen, nämlich umzufällen oder umzukristallisieren. Die Aufarbeitung ist auf einen einzigen Schritt, dem der Fällung in heißem Wasser, verkürzt.

Anschließend wird in der Regel noch weiter mit einem Überschuß an kaltem Wasser verdünnt, wonach filtriert werden kann. Es folgt üblicherweise eine Nachwäsche des Filterkuchens mit heißem und kaltem Wasser. Das hierbei anfallende Waschwasser kann gesammelt und für den Fällungsschritt wiederverwendet werden. Zuletzt kann nach üblichen Methoden, z.B. mit Heißluft, getrocknet werden. Das nach dem erfindungsgemäßen Verfahren erhaltene Produkt ist sehr rein: Eine 2 %ige Lösung in Methylmethacrylat hat mindesten eine Farbzahl < 50; im Regelfall werden aber Farbzahlen < 30 erreicht.

Die Isolierung des Kondensats in einem Schritt reduziert auch Ausbeuteverluste. Es stellen sich durchwegs Ausbeuten über 95 % ein; im Regelfall sogar über 98% bezogen auf den im Unterschuß vorliegenden Reaktanten.

Somit wird durch dieses erfindungsgemäße Merkmal eine Kette von Vorteilen ausgelöst: Die Reaktion findet ausschließlich im wäßrigen Milieu statt, es entfällt die Verwendung organischer Lösungsmittel, das Produkt ist besonders rein, die Ausbeute ist hoch; das Verfahren wird dadurch einfach und wirtschaftlich.

Es ist möglich, das Verfahren kontinuierlich durchzuführen, was den Vorteil hat, kleinvolumiger Reaktionsgefäße verwenden zu können. Die Produktqualität wird dadurch nicht verändert.

Um die niedrigen Farbwerte zu erzielen, sind reine Ausgangssubstanzen selbstverständlich. Vorzugsweise wird beim Einsatz des polymerisierbaren Reaktanden N-Methylol(meth)acrylamid daher auf einen Zusatz zur Stabilisierung des Monomeren, wie z.B. Hydrochinonmonomethyläther, verzichtet. Das gilt für alle Beimengungen, die die Farbzahl erhöhen könnten.

Sublimationsfeste UV-Absorber auf Basis Benztriazol sind schon lange bekannt. Sie zeichnen sich durch einen hohen spezifischen Extinktionskoeffizienten für UV-Licht aus und werden zum Schutz von UV-strahlungsempfindlichen Kunststoffen in diese eingebaut.

Oft werden so modifizierte Polymere auch als Schutzschicht auf die Oberfläche von Kunststoffen aufgebracht.

Das erfindungsgemäße Verfahren kann auch für den Fall angewendet werden, daß in (I) beide Reste aus dem Benztriazolylphenol-Derivat bestehen, daß also in

$$R' - CH_2 - R''$$

R'' durch R' ersetzt ist. Diese symmetrische Verbindung

$$R' - CH_2 - R'$$

wird durch Kondensation eines Benztriazolylphenols mit Formaldehyd, bevorzugt mit Paraformaldehyd, erhalten. Sie kann ebenfalls nach dem erfindungsgemäßen Verfahren hergestellt werden. Man erhält so einen UV-Absorber mit einem erhöhten (verdoppelten) Molekulargewicht, der dadurch schwerflüchtig ist.

Bevorzugt wird das erfindungsgemäße Herstellungsverfahren aber für radikalisch polymerisierbare UV-Absorber angewandt.

Ein Grundbaustein ist Benztriazolylphenol (II).

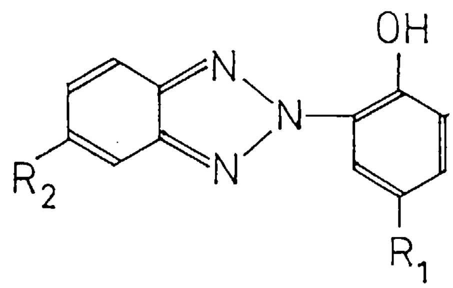

Für die Verbindung (II) in allen aus dem Stand der Technik bekannten chemischen Variationsmöglichkeiten ist das erfindungsgemäße Verfahren unbeschränkt anwendbar. Technisch leicht zugängliche Variationen sind gegeben, wenn der Rest $R_1$ gleich H, $C_1$ - $C_{12}$-Alkyl oder $C_6$ - $C_{10}$-Aryl und der Rest $R_2$ gleich H, Halogen, $C_1$ - $C_{12}$-Alkyl oder $C_6$ - $C_{10}$-Aryl ist. Besonders bevorzugt ist der tert. Octylrest für $R_1$.

Das Benztriazolylderivat wird mit dem radikalisch polymerisierbaren Methylol(meth)acrylamid (III) kondensiert. Die Methacrylamidverbindung ist bevorzugt gegenüber der entsprechenden Acrylamidverbindung, weil sie nach Einpolymerisation zu deutlich härteren Polymeren führt.

BEISPIELE

Beispiel 1

In einem 4 l Glas-Reaktionsgefäß werden 4000 g 98 %ige Schwefelsäure auf 3°C abgekühlt. Unter Rühren und Kühlen werden 725 g 2-(2'-Hydroxy-5'tert.-octylphenyl)-benztriazol zugegeben. Die Temperatur darf dabei nicht über 5 °C ansteigen. Danach werden innerhalb von 3.5 Stunden 490 g feuchtes N-Methylolmethacrylamid (mit einem Wassergehalt von 30 %) zugegeben. Die Temperatur soll möglichst unter 5 °C liegen. Diese Mischung wird innerhalb einer Stunde bei Atmosphärendruck in 8000g siedendes Wasser eingetragen, wobei das Reaktionsprodukt grobteilig ausfällt. Die Siedetemperatur steigt dabei leicht auf 103 °C an. Das heiße Reaktionsgemisch verdünnt und kühlt man durch Zugabe von 20000 g kaltem Wasser. Der Feststoffanteil dieser Suspension wird auf dem Druckfilter abgetrennt. Den Filterkuchen wäscht man 3 mal mit 2100g Kaltwasser und anschließend mit 3600 g Heißwasser. Zuletzt wird getrocknet. Man erhält 929 g Trockensubstanz, was einer Ausbeute von 98.5% bezogen auf das eingesetzte Benztriazolderivat entspricht.
Eine 2 %ige Lösung des trockenen Produktes in Methylmethacrylat hat eine Farbzahl von 28.

Beispiel 2

(Nicht erfindungsgemäße Herstellung: Vergleichsbeispiel)

Es wird wie in Beispiel 1 gearbeitet, mit dem Unterschied, daß

1. die Umsetzungstemperatur bei der Kondensation des Benztriazolderivates mit N-Methylolmethacrylamid auf 25 °C ansteigt, und
2. die Fällung statt in siedendem Wasser in kaltem Wasser erfolgt.

Für das erhaltene Produkt wird eine Farbzahl von 200 gemessen.
Das Produkt ist so nicht brauchbar. Nach dem Stand der Technik müßte also in einem Lösungmittel wie Methylenchlorid oder Chlorbenzol aufgenommen, mit Wasser ausgeschüttelt und eventuell noch umkristallisiert werden, um eine Farbzahl <50 zu erreichen.

**Patentansprüche**

**1.** Verfahren zur Herstellung eines radikalisch polymerisierbaren UV-Absorbers der allgemeinen Formel

$$R' - CH_2 - R'' \qquad (I)$$

wobei R' gleich

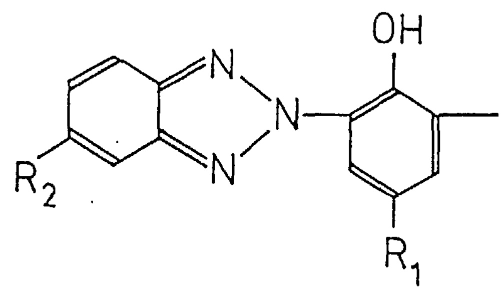

$R_1$ gleich H, $C_1$ - $C_{12}$-Alkyl, $C_6$ - $C_{10}$-Aryl,
$R_2$ gleich H, Halogen, $C_1$ - $C_{12}$-Alkyl, $C_6$ - $C_{10}$-Aryl ist und
R" gleich

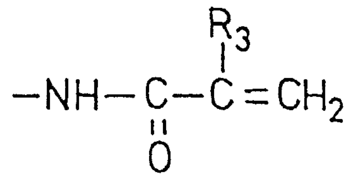

und $R_3$ gleich H oder $CH_3$
ist, durch Kondensation eines Benztriazolylphenols (II)

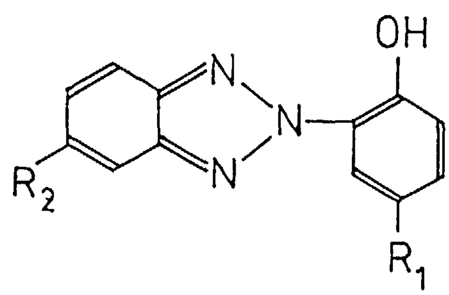

mit N-Methylol(meth)acrylamid (III) in homogener Phase in Schwefelsäure in einer Stufe
dadurch gekennzeichnet, daß

a) die Kondensationsreaktion bei 0-10°C durchgeführt wird
b) die Fällung des in Schwefelsäure gelösten Kondensats durch Eintrag in einen überschuß an Wasser von über 80°C erfolgt.

**2.** Verfahren gemäß Anspruch 1, dadurch gekennzeichnet, daß R1 ein tert. Octylrest ist.

**3.** Verfahren gemäß Anspruch 1 oder 2, dadurch gekennzeichnet, daß die Kondensationreaktion bei 2-5°C durchgeführt wird.

4. Verfahren gemäß einem oder mehreren der Ansprüche 1-3, dadurch gekennzeichnet, daß die Fällung des in Schwefelsäure gelösten Kondensats durch Eintrag in einen überschuß an Wasser von Siedetemperatur erfolgt.

5. Verfahren gemäß einem oder mehreren der Ansprüche 1-4, dadurch gekennzeichnet, daß der Wassergehalt im Kondensationsansatz < 10 Gewichts-% ist.

6. Verfahren gemäß einem oder mehreren der Ansprüche 1-5, dadurch gekennzeichnet, daß der Wassergehalt im Kondensationsansatz < 5 Gewichts-% ist.

7. Verfahren gemäß einem oder mehreren der Ansprüche 1-6, dadurch gekennzeichnet, daß zur Isolierung des in Wasser gefällten Kondensats keine organischen Lösungsmittel verwendet werden.

8. Verfahren gemäß einem oder mehreren der Ansprüche 1-7, dadurch gekennzeichnet, daß das Kondensationsprodukt mit Wasser gewaschen wird, das Waschwasser gesammelt und zur Fällung wiederverwendet wird.

## Claims

1. Process for preparing a radically polymerisable UV absorber of general formula

$$R' - CH_2 - R'' \qquad (I)$$

wherein R' denotes

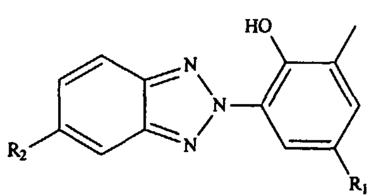

wherein $R_1$ denotes H, $C_{1-12}$-alkyl, $C_{6-10}$-aryl,
$R_2$ denotes H, halogen, $C_{1-12}$-alkyl, $C_{6-10}$-aryl, and R" denotes

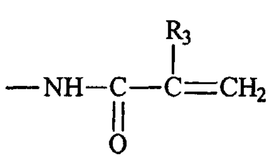

and $R_3$ denotes H or $CH_3$,
by condensation of a benzotriazolylphenol (II)

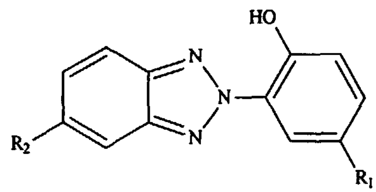

with N-methylol(meth)acrylamide (III) in a homogeneous phase in sulphuric acid, in one step, characterised in that

a) the reaction of condensation is carried out at 0-10°C
b) the precipitation of the condensate dissolved in sulphuric acid is carried out by adding it to an excess of water at over 80°C.

2. Process according to claim 1, characterised in that $R_1$ is a tert.octyl group.

3. Process according to claim 1 or 2, characterised in that the condensation reaction is carried out at 2-5°C.

4. Process according to one or more of claims 1 to 3, characterised in that the precipitation of the condensate dissolved in sulphuric acid is carried out by adding it to an excess of water at boiling temperature.

5. Process according to one or more of claims 1 to 4, characterised in that the water content in the condensation mixture is < 10 wt.%.

6. Process according to one or more of claims 1 to 5, characterised in that the water content in the condensation mixture is < 5 wt.%.

7. Process according to one or more of claims 1 to 6, characterised in that no organic solvents are used to isolate the condensate precipitated in water.

8. Process according to one or more of claims 1 to 7, characterised in that the condensation product is washed with water, the washing water is collected and re-used for the precipitation.

## Revendications

1. Procédé de production d'un absorbant d'UV polymérisable par voie radicalaire, de formule générale

$$R' - CH_2 - R'' \quad (I)$$

dans laquelle R' représente

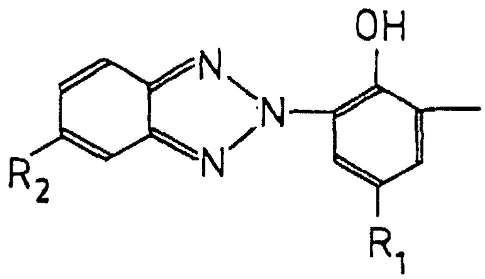

$R_1$ représente H, un alkyle en $C_1$ à $C_{12}$, un aryle en $C_6$ à $C_{10}$,
$R_2$ représente H, un halogène, un alkyle en $C_1$ à $C_{12}$, un aryle en $C_6$ à $C_{10}$ et
R'' représente

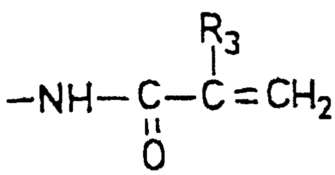

et $R_3$ représente H ou $CH_2$,
par condensation d'un benzotriazolyl phénol (II)

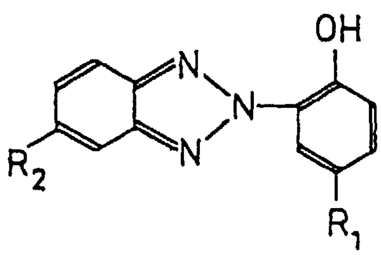

avec du N-méthylol(méth)acrylamide (III) en phase homogène dans l'acide sulfurique en une étape, caractérisé en ce que

a) on conduit la réaction de condensation entre 0 et 10°C,
b) on procède à la précipitation du produit de condensation dissous dans l'acide sulfurique par introduction dans un excès d'eau au-dessus de 80°C.

**2.** Procédé selon la revendication 1, caractérisé en ce que $R_1$ est un radical octyle tertiaire.

**3.** Procédé selon la revendication 1 ou 2, caractérisé en ce qu' on conduit la réaction de condensation entre 2 et 5°C.

**4.** Procédé selon une ou plusieurs des revendications 1 à 3, caractérisé en ce qu' on conduit la précipitation du produit de condensation dissous dans l'acide sulfurique par introduction dans un excès d'eau à la température d'ébullition.

**5.** Procédé selon l'une ou plusieurs des revendications 1 à 4, caractérisé en ce que la teneur en eau dans la charge de condensation est inférieure à 10 % en poids.

**6.** Procédé selon une ou plusieurs des revendications 1 à 5, caractérisé en ce que la teneur en eau dans la charge de condensation est inférieure à 5 % en poids.

**7.** Procédé selon une ou plusieurs des revendications 1 à 6, caractérisé en ce que pour l'isolement du produit de condensation précipité dans l'eau on n'utilise pas de solvant organique.

**8.** Procédé selon une ou plusieurs des revendications 1 à 7, caractérisé en ce qu' on lave le produit de condensation avec de l'eau, en ce qu'on rassemble l'eau de lavage et en ce qu'on la réutilise pour la précipitation.